# EUROPÄISCHE PATENTSCHRIFT

(11) **EP 1 149 606 B1**
(45) Veröffentlichungstag und Bekanntmachung des Hinweises auf die Patenterteilung: **23.05.2007**
(21) Anmeldenummer: 01109752.4
(22) Anmeldetag: 20.04.2001
(51) Int. Cl.: A63B 23/02, A63B 24/00

(54) **Rückenstützvorrichtung**
Back support apparatus
Dispositif de support dorsal

(30) Priorität: 25.04.2000 DE 10020248
(43) Veröffentlichungstag der Anmeldung: 31.10.2001
(73) Patentinhaber: Berschin, Gereon, Dr., 35091 Cölbe (DE)
(72) Erfinder: Berschin, Gereon, Dr., 35091 Cölbe (DE); Sommer, Hans-Martin, Prof.Dr.med., 69115 Heidelberg (DE); Rohrscheidt-Sommer, Charlotte, 69115 Heidelberg (DE)
(74) Vertreter: Olbricht, Karl Heinrich

(56) Entgegenhaltungen:
- EP-A- 0 993 845
- WO-A-94/10877
- DE-A- 19 608 098
- FR-A- 2 750 341
- US-A- 5 356 353
- US-A- 5 755 647

## Beschreibung

Die Erfindung betrifft eine Rückenstützvorrichtung gemäß dem Oberbegriff von Anspruch 1.

Beim Muskeltraining beispielsweise auf einer Trainingsliege unter Einsatz von Zusatzlasten besteht oft das Problem, daß die Wirbelsäule unbeachtet in eine Position gerät, die auf Dauer zu körperlichen Beschwerden oder gar Schäden führen kann. Um dem zu begegnen, hat man mit mechanischen Hilfsmitteln versucht, das Problem einer Fehlbewegung oder Fehlhaltung zu vermeiden. So sind Trainingsgeräte mit Rückenstützvorrichtungen bekannt, die mittels einer anatomischen Polsterung oder verstellbaren Barrieren oder durch Festschnallen der Hüfte versuchen, die Wirbelsäule in eine schonende Haltung zu zwingen, was jedoch nur eingeschränkt gelingen kann. Durch einen meist unbewußt entstehenden aktiven Druck gegen einen blockierenden Widerstand können Schäden entstehen.

Bei einer aus DE-A1-41 39 241 bekannten Vorrichtung mit einem Patientengerät und einer optisch-akustischen Anzeigeeinrichtung werden Sollwerte von Kontraktionskraft und -dauer von zu behandelnden Muskelpartien vor Beginn eines Patiententrainings festgelegt; das Erreichen dieser Sollwerte wird mit Hilfe der Anzeigeeinrichtung über einen gewissen Zeitraum geübt. DE-A1-195 OS 735 berücksichtigt zusätzlich die Kontraktionsdauer und den Ruhetonus der Muskulatur, um Über- und Unterbelastungen zu vermeiden. Eine andere in DE-A1-41 31 422 beschriebene Vorrichtung verwendet beim Krafttraining mit Gewichten ebenfalls Meß- und/oder Anzeigeeinrichtungen zum Erzeugen eines akustischen und/oder optischen Signals bei Überschreiten einer durch den menschlichen Körper ausgeübten Druckkraft, damit einer Überlastung der Muskulatur vorgebeugt werden kann.

Eine ähnliche Problematik ergibt sich beim Ausüben von sitzender Tätigkeit, beispielsweise auf einem Bürostuhl. In der Druckschrift WO 94/10877, gegenüber welcher Anspruch 1 abgegrenzt ist, ist ein ergonomisch gestalteter Sitz insbesondere als Drehstuhl beschrieben, bei dem die Sitzfläche und eine höhenverstellbare, annähernd anatomisch geformte Rückenlehne mit Drucksensoren ausgestattet sind, welche die Sitzbelastung sowie den Rücken-Andruck vor allem im Bereich der Lendenwirbel erfassen und digital über einen Mikroprozessor auswerten. Das Verhältnis der Meßwerte kann in Abhängigkeit von gespeicherten Vorgaben akustische Signale auslösen, die zur Korrektur der Sitzposition auffordern oder diese motorisch bewirken. Hier wie bei Konstruktionen mit Synchron-Federungenversucht man, den Körper und damit die Wirbelsäule in eine vermeintlich natürliche Position zu bringen, auf Dauer oft nur mit mäßigem Erfolg.

Man hat auch Einrichtungen vorgeschlagen, die mit akustischen und/oder optischen Anzeigegeräten die gezielte Stärkung bestimmter Muskelgruppen unter Biofeedback anstreben, wobei eine Positionsüberwachung von Körperteilen jedoch allgemein nicht stattfindet. Mit Biofeedback-Methoden werden physiologische Abläufe, die nicht oder nur sehr ungenau durch Sinnesorgane erfaßt werden, der bewußten Wahrnehmung zugänglich gemacht. Damit lassen sich auch Funktionen gezielt beeinflussen, die für eine exakte und unmittelbare Wahrnehmung der ablaufenden Prozesse eine Voraussetzung sind.

Der vorliegenden Erfindung liegt die Aufgabe zugrunde, eine ständige Kontrolle der Wirbelsäulenpositionunter Biofeedback zu verbessern, um beispielsweise im Liegen oder bei sitzender Tätigkeit ein Aufbautraining zu ermöglichen. Eine zu schaffende Rückenstützvorrichtungsoll insbesondere im Bereich der Lendenwirbel wirksam und im Übrigen einfach aufgebaut, kostengünstig herstellbar und leicht zu handhaben sein.

Hauptmerkmale der Erfindung sind in Anspruch 1 angegeben. Ausgestaltungen sind Gegenstand der Ansprüche 2 bis 15.

Bei einer Rückenstützvorrichtung zum Überwachen und Biofeedback der Position einer Wirbelsäule beim Muskeltraining oder bei Ausübung einer sitzenden Tätigkeit einer Person, mit einer Rückenauflagefläche und einer in dieser integrierten Meßvorrichtung zur Erfassung der Wirbetsäulen-Position relativ zu der Rückenauflagefläche, wobei die Meßvorrichtung wenigstens einen Meßwertaufnehmer aufweist, der mit einem Mikroprozessor verbunden ist, um Positions-Istwerte mit vorgegebenen Positions-Sollwerten zu vergleichen, und wobei ein z.B. akustisches Signal abgebbar ist, falls die Istwerte außerhalb der Sollwerte liegen, ist laut Anspruch 1 erfindungsgemäß vorgesehen, daß der wenigstens eine Meßwertaufnehmer innerhalb der Rückenauflagefläche in wenigstens zwei Raumrichtungen relativ zur Wirbelsäule verstellbar sowie im Kontakt mit dieser in einer vorgebbaren Position fixierbar ist und daß wenigstens eine Anzeigevorrichtung vorhanden ist, mittels derer ein optisches und/oder akustisches und/oder taktiles Signal abgebbar ist, wenn ein Positions-Sollwert und/oder ein geometrischer und/oder zeitlicher Toleranzbereich für die Positions-Sollwerte des Wirbelsäule verlassen wird.

Gerade beim Krafttraining ist eine gezielte individuelle Überwachung und ständige Beobachtung der Wirbelsäulenhaltung wichtig, da diese bei der Verwendung von Gewichten oder Lasten zu Ausweichbewegungen neigt, die mit Sinnesorganen nicht oder nur unzureichend wahrnehmbar sind. Durch systematische Steigerung der Gewichtskraft in herkömmlicher Weise kann zwar der Trainingseffekt erhöht werden, jedoch mit der Nebenwirkung, daß sich Schäden an der Wirbelsäule ausbilden können. Dies wird erfindungsgemäß auf einfache und effektive Weise durch Biofeedback zuverlässig vermieden, indem zumindest ein in wenigstens zwei - vorzugsweise drei - Raumrichtungen relativ zur Wirbelsäule verstellbarer und im Kontakt mit ihr in einer vorgebbaren Position fixierbarer Meßwertaufnehmer die IstPosition der Wirbelsäule erfaßt. Mittels einer Eingabeeinheit lassen sich die Soll- und Toleranzbereichswerte, die aus den von der Wirbelsäule der trainierenden Person erreichbaren Extremstellungen ermittelt oder errechnet werden, und die gemessenen Istwerte in einer Speichereinheit festhalten. Jeder Meßwertaufnehmer ist vor dem Trainingsbeginn kalibrierbar. Die ermittelte Istposition wird in einer Auswerteelektronik mikroprozessorgesteuert mit vorgegebenen Sollwerten verglichen und es wird ein optisches, akustisches und/oder taktiles Signal ausgelöst, sobald ein geometrisch und/oder zeitlich festlegbarer Toleranzbereich der Sollwerte überschritten wird.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Fig. 1: eine schematische Seitenansicht einer Trainingsliege, teilweise im Schnitt,
- Fig. 2: eine Draufsicht auf die Trainingsliege von Fig. 1 und
- Fig. 3: ein Blockschaltbild einer elektronischen Auswerteschaltung.

Gerade beim Krafttraining ist jedoch eine gezielte und individuelle Überwachung und Wahrnehmung der Wirbelsäulenhaltung wichtig, da die Wirbelsäule bei der Verwendung von Gewichten oder Lasten zu Ausweichbewegungen neigt, die von den Sinnesorganen nicht oder nur unzureichend wahrnehmbar sind. Folglich kann bei einer systematischen Steigerung der Gewichtskraft zwar der Trainingseffekt erhöht werden, jedoch mit dem Nebeneffekt, daß sich Schäden an der Wirbelsäule ausbilden können. Dies wird durch die Erfindung auf einfache und effektive Weise zuverlässig vermieden.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:

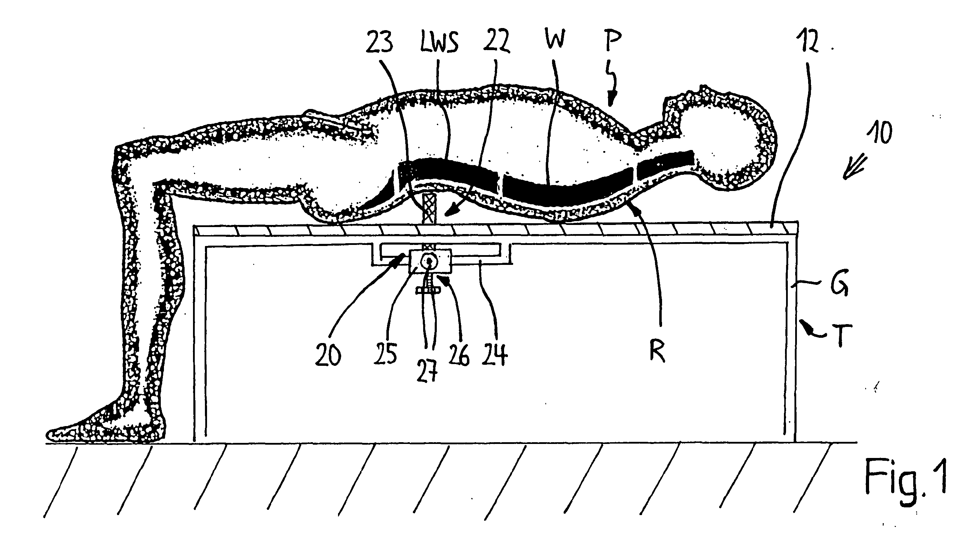

- Fig. 1: eine schematische Seitenansicht einer Trainingsliege, teilweise im Schnitt,

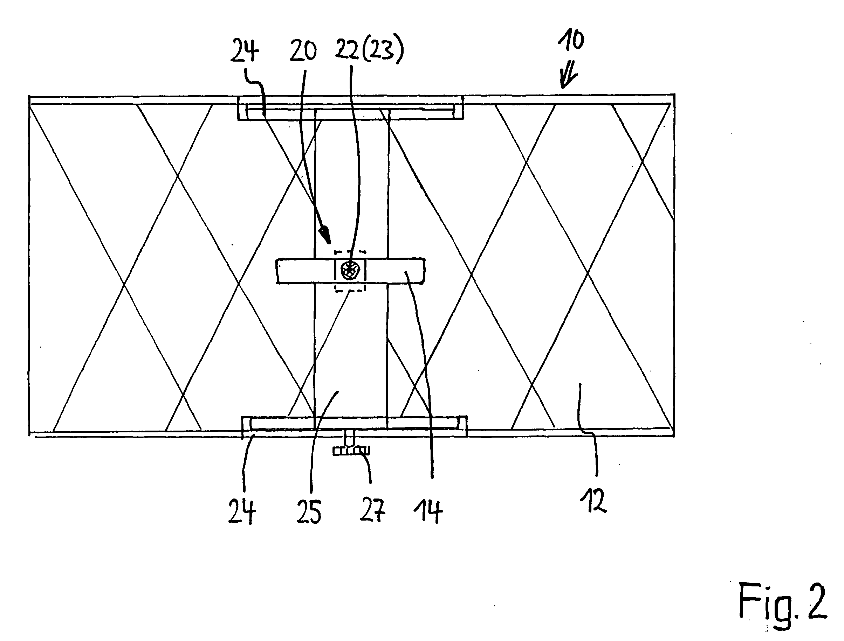

- Fig. 2: eine Draufsicht auf die Trainingsliege von Fig. 1 und

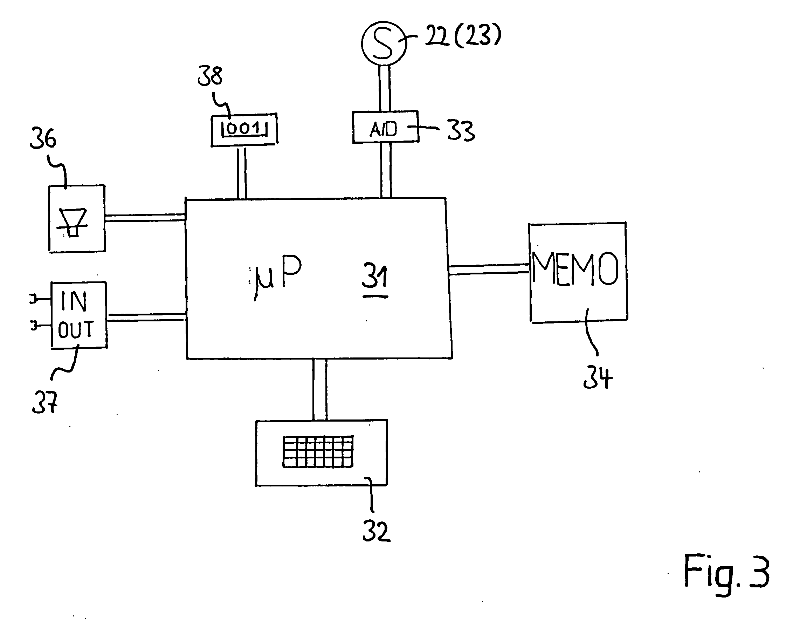

- Fig. 3: ein Blockschaltbild einer elektronischen Auswerteschaltung.

Die in Fig. 1 allgemein mit 10 bezeichnete Rückenstützvorrichtung ist Bestandteil einer Trainingsliege T für das Krafttraining. Letztere hat ein Rahmengestell G, auf dem eine Rückenauflagefläche 12 in Form einer Liegefläche angebracht ist. Auf der Liegefläche 12 liegt ein Patient P mit dem Rücken R auf, wobei die Wirbelsäule W sich in Längsrichtung der Liegefläche 12 erstreckt. Letztere ist etwa in der Mitte durch eine längsschlitzförmige Aussparung 14 unterbrochen (siehe Fig. 2).

Unter der Rückenauflagefläche 12, die bei Bedarf abschnittsweise anatomisch geformt sein kann, sind beispielsweise am Rahmengestell G zwei Führungsrohre 24 befestigt, auf denen längsbeweglich ein Schlitten 25 gelagert ist. In der Mitte des Schlittens 25 ist eine Meßvorrichtung 20 mit einem Meßwertaufnehmer 22 befestigt, der mit Hilfe einer Höhenjustierung 26 in seiner Vertikalposition relativ zur Wirbelsäule W der trainierenden Person P verändert werden kann. Für die Positionierung des Meßwertaufnehmers 22 quer zur Liegefläche 12, kann eine zusätzliche (nicht dargestellte) seitliche Verstelleinrichtung vorgesehen sein. Sowohl der Schlitten 25 als auch die Höhenjustierung 26 lassen sich mittels Stellschrauben 27 rasch und bequem in ihren festzulegenden Positionen fixieren.

Der Meßwertaufnehmer 22 ist in der Ausführungsform von Fig. 1 ein kapazitiver Positionssensor mit einem Taststift 23. Dieser wird mittels des Schlittens 25 und der Höhenjustierung 26 vor Beginn des Trainings derart positioniert, daß zwischen den Extremstellungen der Wirbelsäule W des Trainierenden im Lendenwirbelbereich LWS ein ständiger Kontakt zwischen Rücken und Taststift 23 gewährleistet ist. Bewegungen der Wirbelsäule W während des Trainings werden folglich, ausgehend von einer Ausgangsstellung, in der die Wirbelsäule W optimal entspannt ist, stets unmittelbar auf den Taststift 23 übertragen und von dem Meßwertaufnehmer 22 und damit von der Meßvorrichtung 20 erfaßt.

Letztere liefert die Istposition der Wirbelsäule W an eine in Fig. 3 gezeigte elektronische Auswerteschaltung 30. Diese besitzt einen Mikroprozessor 31, der die mittels eines A/D-Wandlers 33 an den Mikroprozessor 31 übermittelten Positions-Istwerte der Wirbelsäule W mit in einem Speicher 34 gespeicherten Sollwerten vergleicht. Für die Vorgabe der Sollwerte 34 ist eine Eingabevorrichtung 32, beispielsweise eine Tastatur vorgesehen. Man kann die Sollwerte 34 aber auch über eine Schnittstelle 37 der Auswerteschaltung 30 zuleiten.

Neben den von der Wirbelsäule W während des Trainings einzuhaltenden Sollwerten werden Toleranzbereiche festgelegt und mit der Eingabevorrichtung 32 in den Speicher 34 eingegeben. Die Vorgabewerte der Toleranzbereiche umfassen zum einen geometrische Angaben, die sich auf die Bewegung des Taststifts 23 beziehen. Zum anderen umfassen die Vorgabewerte Zeitangaben, innerhalb denen sich der Taststift 23 außerhalb der geometrischen Toleranzvorgaben befinden darf. Letzteres hat für den Trainierenden den Vorteil, daß nicht gleich jede Fehlbewegung zur Alarmauslösung führt.

Man erkennt, daß während des Trainings jede Bewegung der Wirbelsäule W von dem Positionssensor 22, 23 erfaßt und an die Auswerteelektronik 30 weitergeleitet wird. Diese vergleicht mittels des Mikroprozessors 31 jeden Positions-Istwert anhand der Toleranzwerte mit den ebenfalls im Speicher 34 abgelegten Sollwerten und informiert den Trainierenden über einen Signalgeber 36 unmittelbar, wenn die Wirbelsäule W ihre Sollposition verlassen und die vorgegebenen Toleranzwerte überschritten hat. Der Trainierende kann sofort seine Haltung korrigieren, wobei der Signalgeber 36 abgeschaltete wird, sobald die Wirbelsäule W wieder ihre optimale Position eingenommen hat.

Vor jedem Einsatz muß die Meßvorrichtung 20 und jeder Positionssensor 22, 23 individuell an den Trainierenden angepaßt und die Auswerteelektronik 30 entsprechend der Beweglichkeit der Wirbelsäule W kalibriert werden. Sobald diese eine für das gewünschte Training geeignete Haltung inne hat, wird der Meßwertaufnehmer 22 mit Hilfe der horizontalen Verstelleinrichtungen 24, 25 so eingestellt, daß der Taststift 23 an der gewünschten Stelle am Rücken R bzw. der Wirbelsäule W angreift. Die Höhenjustierung 26 wird hingegen so fixiert, daß der Taststift 23 in der Ruhestellung der Wirbelsäule W in seiner Nullstellung liegt. Anschließend wird zunächst die Wirbelsäule W maximal gegen die Liegefläche 12 gedrückt und danach maximal von der Liegefläche 12 abgehoben. Die auf diese Weise ermittelten Positionswerte werden als Kalibrierwerte im Speicher 34 der Auswerteelektronik 30 abgelegt. Unter Zuhilfenahme dieser Kalibrierwerte wird dann der Sollwert und der zulässige Toleranzbereich für die Rückenposition festgelegt. Die Größe der Toleranzbereiche richten sich nach dem Trainingszustand des Trainierenden. Man kann für die Toleranzbereiche aber auch Tabellenwerte festlegen, die anhand orthopädischer und/oder anatomischer Voruntersuchungen gewonnen werden. Sämtliche geometrischen und zeitlichen Toleranzwerte werden über die Eingabeeinheit 32 eingegeben und ebenfalls gespeichert.

Der Positionssensor 22, 23 kann nun während des Trainings die individuell erforderliche Position des Trainierenden überwachen. Verläßt dessen Wirbelsäule W die vorgegebene Sollposition, wird dies über die Anzeigeeinrichtung 36 akustisch und/oder optisch angezeigt. Die Haltung kann sofort korrigiert werden. Dieser Zustand ist dann erreicht, wenn die Anzeigeeinrichtung 36 verstummt.

Die Erfindung ist nicht auf eine der vorbeschriebenen Ausführungsformen beschränkt, sondern in vielfältiger Weise abwandelbar. So können innerhalb der Rückenauflagefläche 12 mehrere Meßwertaufnehmer 22 angeordnet werden, um beispielsweise die Wirbelsäule W über ihre gesamte Länge zu überwachen, wobei man als Meßwertaufnehmer auch Drucksensoren verwenden kann. Um die Anzahl oder die Häufigkeit der Abweichungen von der Sollposition zu erfassen, wird an den Mikroprozessor 31 ein Zählwerk 38 angeschlossen, dessen Werte bei Bedarf ebenfalls in der Speichereinheit 34 gespeichert werden können. Der gesamte Inhalt der Speichereinheit 34 kann über die Schnittstelle 37 ausgelesen und zur weiteren Analyse beispielsweise mittels eines (nicht dargestellten) PCs ausgewertet werden. Die Überwachung der Position kann im Bereich der Lendenwirbelsäule (LWS) erfolgen, doch ist parallel dazu auch eine Erfassung des Hüftwinkels möglich.

In einer anderen (nicht dargestellten) Ausführungsform der Erfindung ist die Rückenstützvorrichtung 10 in einer Sitzvorrichtung, z.B. einem Bürostuhl, ausgebildet. Die sitzende Person liegt mit ihrem Rücken flach an einer Rückenlehne an, in der die Rückenstützvorrichtung 10 integriert ist. Auf diese Weise wird während des Sitzens und damit während der Ausübung einer Tätigkeit ständig die Rückenhaltung überwacht. Sobald die Wirbelsäule W eine gekrümmte Haltung einnimmt, wird über die Auswerteschaltung 30 und deren Signalgeber 36 ein Warnsignal abgegeben.

Man erkennt, daß beim Biofeedback-Training zum Überwachen der Position einer Wirbelsäule W beim Muskeltraining oder bei der Ausübung einer sitzenden Tätigkeit erfindungsgemäß vorgesehen ist, daß ein in wenigstens zwei Raumrichtungen relativ zur Wirbelsäule W verstellbarer und im Kontakt mit dieser in einer vorgebbaren Position fixierbarer Meßwertaufnehmer 22 die Istposition der Wirbelsäule W permanent erfaßt und daß die auf diese Weise ermittelte Istposition in einer mikroprozessorgesteuerten elektronischen Auswerteschaltung 30 mit einem vorgegebenen Sollwert verglichen wird, wobei außerhalb eines vorgebbaren geometrischen und/oder zeitlich festlegbaren Toleranzbereichs ein optisches, akustisches und/oder taktiles Signal ausgelöst wird. Sämtliche Ist-, Soll- und Vorgabewerte sind in einer Speichereinheit 34 speicherbar und bei Bedarf zur Auswertung abrufbar

### Bezugszeichenliste

- G: Rahmengestell
- P: Person
- T: Trainingsliege
- W: Wirbelsäule

- 10: Rückenstützvorrichtung
- 12: Rückenauflagefläche
- 14: Aussparung

- 20: Meßvorrichtung
- 22: Meßwertaufnehmer
- 23: Taststift
- 24: Führungsrohre
- 25: Schlitten
- 26: Höhenjustierung
- 27: Stellschrauben

- 30: Auswerteschaltung
- 31: Mikroprozessor
- 32: Eingabeeinheit
- 33: A/D-Wandler
- 34: Speichereinheit
- 36: Anzeigevorrichtung
- 37: Schnittstelle
- 38: Zählwerk

## Patentansprüche

1. Rückenstützvorrichtung (10) zum Überwachen und Biofeedback der Position einer Wirbelsäule (W) beim Muskeltraining oder bei Ausübung einer sitzenden Tätigkeit einer Person (P), mit einer Rückenauflagefläche (12) und einer in dieser integrierten Meßvorrichtung (20) zur Erfassung der Wirbelsäulen-Position relativ zu der Rückenauflagefläche (12), wobei die Meßvorrichtung (20) wenigstens einen Meßwertaufnehmer (22) aufweist, der mit einem Mikroprozessor (31) verbunden ist, um Positions-Istwerte mit vorgegebenen Positions-Sollwerten zu vergleichen, und wobei ein z.B. akustisches Signal abgebbar ist, falls die Istwerte außerhalb der Sollwerte liegen, **dadurch gekennzeichnet, daß** der wenigstens eine Meßwertaufnehmer (22) innerhalb der Rückenauflagefläche (12) in wenigstens zwei Raumrichtungen relativ zur Wirbelsäule (W) verstellbar sowie im Kontakt mit dieser in einer vorgebbaren Position fixierbar ist und daß wenigstens eine Anzeigevorrichtung (36) vorhanden ist, mittels derer ein optisches und/oder akustisches und/oder taktiles Signal abgebbar ist, wenn der Positions-Sollwert und/oder ein geometrischer und/oder zeitlicher Toleranzbereich für die Positions-Sollwerte der Wirbelsäule (W) verlassen wird.

2. Rückenstützvorrichtung nach Anspruch 1, **dadurch gekennzeichnet, daß** mit dem Mikroprozessor (31) eine Auswerteelektronik (30) verbunden ist, die eine Eingabeeinheit (32), eine Speichereinheit (34) und die wenigstens eine Anzeigevorrichtung (36) aufweist.

3. Rückenstützvorrichtung nach Anspruch 1 oder 2, **dadurch gekennzeichnet, daß** in der Rückenauflagefläche (12) wenigstens eine Aussparung (14) vorgesehen ist, innerhalb derer der Meßwertaufnehmer (22) verstellbar angeordnet ist.

4. Rückenstützvorrichtung nach einem der Ansprüche 1 bis 3, **dadurch gekennzeichnet, daß** die Rückenauflagefläche (12) ein- oder mehrteilig verstellbar ausgebildet ist.

5. Rückenstützvorrichtung nach einem der Ansprüche 1 bis 4, **dadurch gekennzeichnet, daß** die Rückenauflagefläche (12) anatomisch vorgeformt oder zumindest abschnittsweise anatomisch verformbar ist.

6. Rückenstützvorrichtung nach einem der Ansprüche 1 bis 5, **dadurch gekennzeichnet, daß** zumindest einer der Meßwertaufnehmer (22) gegenüber der Lendenwirbelsäule (LWS) in drei voneinander unabhängigen Richtungen verstellbar ist.

7. Rückenstützvorrichtung nach einem der Ansprüche 1 bis 6, **dadurch gekennzeichnet, daß** zumindest einer der Meßwertaufnehmer (22) motorisch verstellbar ist.

8. Rückenstützvorrichtung nach einem der Ansprüche 1 bis 7, **dadurch gekennzeichnet, daß** zumindest einer der Meßwertaufnehmer (22) die Position der Wirbelsäule (W) dorsal erfaßt.

9. Rückenstützvorrichtung nach einem der Ansprüche 1 bis 8, **dadurch gekennzeichnet, daß** der oder jeder Meßwertaufnehmer (22) ein Positionssensor oder ein Drucksensor ist.

10. Rückenstützvorrichtung nach einem der Ansprüche 1 bis 9, **dadurch gekennzeichnet, daß** dem Mikroprozessor (31) ein Zeitgeber oder ein Zählwerk (38) zugeordnet ist.

11. Rückenstützvorrichtung nach einem der Ansprüche 2 bis 10, **dadurch gekennzeichnet, daß** die Positions-Sollwerte der Wirbelsäule (W) und/oder der geometrische und/oder zeitliche Toleranzbereich für die Positions-Sollwerte sowie die von dem oder jedem Meßwertaufnehmer (22) erfaßten Positions-Istwerte in der Speichereinheit (34) speicherbar sind.

12. Rückenstützvorrichtung nach einem der Ansprüche 2 bis 11, **dadurch gekennzeichnet, daß** die Anzeigevorrichtung (36) eine LCD-Kette oder ein LCD-Display ist.

13. Rückenstützvorrichtung nach einem der Ansprüche 2 bis 11, **dadurch gekennzeichnet, daß** die Anzeigevorrichtung (36) ein Tongeber ist.

14. Sitzvorrichtung mit einer Rückenstützvorrichtung nach einem der Ansprüche 1 bis 13.

15. Trainingsvorrichtung mit einer Rückenstützvorrichtung nach einem der Ansprüche 1 bis 13.

## Claims

1. Dorsal support device (10) to monitor and biofeedback the position of a spine (W) of a person (P) when exercising muscle training or a sitting activity, with a dorsal support surface (12) and a therein integrated measuring device (20) to register the spine position relatively to the dorsal support surface (12), with such measuring device (20) being equipped with at least one force meter (22) connected to a microprocessor (31) to compare actual position values with given set position values and emitting for example an acoustic signal when the actual values are beyond the set values, **characterized by** the at least one force meter (22) within the dorsal support surface (12) being adjustable in at least two spatial directions relatively to the spine (W) and in contact with the same fixable in a given position, and by being equipped with at least one display device (36) able to emit an optical and/or acoustic and/or tactile signal when the set position value and/or a geometric and/or a temporal range of tolerance for the set position values of the spine (W) is left.

2. Dorsal support device according to claim 1, **characterized by** an electronic evaluation device (30) being linked to the microprocessor (31) and showing an input unit (32), a storage unit (34) and at least one display device (36).

3. Dorsal support device according to claims 1 or 2, **characterized by** the dorsal support surface providing for at least one recess (14) wherein the force meter (22) is adjustably disposed.

4. Dorsal support device according to one of the claims 1 to 3, **characterized by** the dorsal support surface (12) is executed to be adjustable in one or several parts.

5. Dorsal support device according to one of the claims 1 to 4, **characterized by** the dorsal support surface (12) is anatomically preformed or at least anatomically deformable in sections.

6. Dorsal support device according to one of the claims 1 to 5, **characterized by** at least one of the force meters (22) being adjustable in three directions independent from each other, relatively to the lumbar vertebrae (LWS).

7. Dorsal support device according to one of the claims 1 to 6, **characterized by** at least one of the force meters (22) is adjustable by motor.

8. Dorsal support device according to one of the claims 1 to 7, **characterized by** at least one of the force meters (22.) registers the position of the spine (W) dorsally.

9. Dorsal support device according to one of the claims 1 to 8, **characterized by** one or each of the force meters (22) being a position detector or a pressure detector.

10. Dorsal support device according to one of the claims 1 to 9, **characterized by** a timer or a meter (38) is associated to the microprocessor (31).

11. Dorsal support device according to one of the claims 2 to 10, **characterized by** the set position values of the spine (W) and/or the geometric and/or temporal range of tolerance for the set position values as well as the actual position values registered by the or each force meter (22) being storable in the storage unit (34).

12. Dorsal support device according to one of the claims 2 to 11, **characterized by** the display device (36) being an LC-chain or an LC-display.

13. Dorsal support device according to one of the claims 2 to 11, **characterized by** the display device (36) being a sound emitter.

14. Seat comprising a dorsal support device according to one of the claims 1 to 13.

15. Training device comprising a dorsal support device according to one of the claims 1 to 13.

## Revendications

1. Dispositif d'appui dorsal (10) destiné au contrôle et au biofeedback de la position d'une colonne vertébrale (W) lors d'exercices de musculation ou lors de l'exercice d'une activité assise d'une personne (P), comprenant une surface d'appui dorsal (12) et un dispositif de mesure (20) intégré dans celle-ci, destiné à enregistrer la position de la colonne vertébrale par rapport à la surface d'appui dorsal (12), le dispositif de mesure (20) présentant au moins un capteur de valeurs de mesure (22) raccordé à un microprocesseur (31), afin de comparer des valeurs réelles de positions à des valeurs théoriques de positions prédéfinies, et un signal acoustique, par ex., pouvant être donné au cas où les valeurs réelles soient situées en dehors des valeurs théoriques, **caractérisé en ce que** l'au moins un capteur de valeurs de mesure (22) est réglable, dans la surface d'appui dorsal (12), dans au moins deux directions spatiales par rapport à la colonne vertébrale (W) et qu'il peut être fixé, en étant en contact avec celle-ci, dans une position pouvant être prédéfinie et **en ce qu'**au moins un dispositif de visualisation (36) est disponible, au moyen duquel un signal optique et/ou acoustique et/ou tactile peut être donné lorsque la valeur théorique de la position et/ou une plage de tolérance géométrique et/ou une plage de tolérance de temps pour les valeurs théoriques de positions de la colonne vertébrale (W) sont quittées.

2. Dispositif d'appui dorsal selon la revendication 1, **caractérisé en ce qu'**un dispositif d'évaluation électronique (30) est raccordé au microprocesseur (31), ce dispositif d'évaluation électronique présentant une unité d'entrée (32), une unité de mémoire (34) et au moins un dispositif de visualisation (36).

3. Dispositif d'appui dorsal selon la revendication 1 ou 2, **caractérisé en ce qu'**au moins un évidement (14) est prévu dans la surface d'appui dorsal (12), dans lequel est disposé le capteur de valeurs de mesure (22) de manière réglable.

4. Dispositif d'appui dorsal selon l'une quelconque des revendications 1 à 3, **caractérisé en ce que** le dispositif d'appui dorsal (12) est exécuté de manière réglable en une seule partie ou en plusieurs parties.

5. Dispositif d'appui dorsal selon l'une quelconque des revendications 1 à 4, **caractérisé en ce que** le dispositif d'appui dorsal (12) est préformé anatomiquement ou est déformable anatomiquement au moins par sections.

6. Dispositif d'appui dorsal selon l'une quelconque des revendications 1 à 5, **caractérisé en ce qu'**au moins l'un des capteurs de valeurs de mesure (22) est réglable dans trois directions, indépendantes l'une de l'autre, par rapport aux vertèbres lombaires (LWS).

7. Dispositif d'appui dorsal selon l'une quelconque des revendications 1 à 6, **caractérisé en ce qu'**au moins l'un des capteurs de valeurs de mesure (22) est réglable par moteur.

8. Dispositif d'appui dorsal selon l'une quelconque des revendications 1 à 7, **caractérisé en ce qu'**au moins l'un des capteurs de valeurs de mesure (22) enregistre la position de la colonne vertébrale (W) de manière dorsale.

9. Dispositif d'appui dorsal selon l'une quelconque des revendications 1 à 8, **caractérisé en ce que** le capteur de valeurs de mesure ou chaque capteur de valeurs de mesure (22) est un capteur de position ou un capteur de pression.

10. Dispositif d'appui dorsal selon l'une quelconque des revendications 1 à 9, **caractérisé en ce qu'**un temporisateur ou un compteur (38) est associé au microprocesseur (31).

11. Dispositif d'appui dorsal selon l'une quelconque des revendications 2 à 10, **caractérisé en ce que** les valeurs théoriques de positions de la colonne vertébrale (W) et/ou la plage de tolérance géométrique et/ou la plage de tolérance de temps pour les valeurs théoriques de positions ainsi que les valeurs réelles de positions enregistrées par le capteur de valeurs de mesure ou par chaque capteur de valeurs de mesure sont mémorisables dans l'unité de mémoire (34).

12. Dispositif d'appui dorsal selon l'une quelconque des revendications 2 à 11, **caractérisé en ce que** le dispositif de visualisation (36) est une chaîne à cristaux liquides ou un écran à cristaux liquides.

13. Dispositif d'appui dorsal selon l'une quelconque des revendications 2 à 11, **caractérisé en ce que** le dispositif de visualisation (36) est un générateur de tonalité.

14. Siège comprenant un dispositif d'appui dorsal selon l'une quelconque des revendications 1 à 13.

15. Dispositif d'entraînement comprenant un dispositif d'appui dorsal selon l'une quelconque des revendications 1 à 13.
